# EUROPEAN PATENT APPLICATION

(11) **EP 4 759 222 A1**
(43) Date of publication of application: **17.06.2026**
(21) Application number: 24220350.3
(22) Date of filing: 16.12.2024
(51) Int. Cl.: A61B 1/005, A61B 1/00, A61B 1/05, A61B 1/008

(54) **ENDOSCOPE WITH A BENDING SECTION CONTROLLED BY STEERING CABLES**

(71) Applicant: Ambu A/S, 2750 Ballerup (DK)
(72) Inventor: HANSEN, Frederik Clausager Vemb, 2400 København NV (DK)
(74) Representative: Winter, Brandl - Partnerschaft mbB

(57) **Abstract**

An endoscope (2) and a visualization system (21) including the endoscope (2), the endoscope (2) including a proximal end (6p) and a distal end (6d) spaced apart from the proximal end (6p); a camera (30) and a light source (32) positioned at the distal end (6d); and a bending section (10) controllable by steering cables made up of steering wire (44) and wire pipe (46). A proximal segment (6p) of the bending section (10) has a wire pipe abutment (58) arranged with a spacing from a horizontal midplane (60) of the bending section (10).

## Description

### TECHNICAL FIELD

The present disclosure relates to an endoscope with a bending section, especially a bending section with a wire pipe abutment at the proximal end, and a visualization system comprising the endoscope and video processing apparatus.

### BACKGROUND

Endoscopes and visualization systems including video processing apparatus electrically connected to an endoscope are known and can be used for visual navigation into, and examination and diagnosis of, hollow organs and body cavities, as well as, optionally, to assist in surgery, e.g. for a targeted tissue sampling. Endoscopes include procedure-specialized endoscopes, such as bronchoscopes, arthroscopes, cystoscopes, ureteroscopes, cholangioscopes, colonoscopes, laparoscopes, gastroscopes, and duodenoscopes. A visualization system including an endoscope is described in commonly-owned U.S. Patent No. 10,646,107. A visualization system including a portable medical monitor having a display screen is described in commonly-owned U.S. Patents Nos. 11,266,297 and 11,328,390.

Single-use endoscopes optimize workflow and reduce cost while saving patient's lives and improving patient care. They optimize workflow and reduce cost because they are always ready when needed without the traditional large-scale capital and repair budgets required for reusable intrusive medical devices. For example, a sterilization and storage facility is avoided, there is no need to maintain evidence of sterilization, and there is no need to transport endoscopes from sterilization and storage facilities to the buildings where they are needed, sometimes in the middle of the night or weekends. They save patient's lives and improve patient care because they are readily available and do not pose a cross-contamination risk. This also reduces hospital re-admissions. While single-use endoscopes are disposed after a single patient use (one or more procedures may be performed while the patient remains in the treatment room), the environmental impact of re-useable endoscopes, due to cleaning materials, CO₂ emissions during the cleaning process, and use of disposable personal protective equipment by personnel involved in transportation and sterilization of the re-useable endoscopes, can be similar to that of single-use endoscopes. To further reduce environmental impact, the endoscope according to the present disclosure is primarily made of polymer materials.

To further enhance the benefits of single-use endoscopes, it is desirable to improve the function thereof.

To further enhance the benefits of single-use endoscopes, it is desirable to reduce manufacturing costs.

### SUMMARY

The present disclosure provides an endoscope with a bending section having a wire pipe abutment arranged with a spacing from a horizontal midplane of the bending section. Hereby available space in an insertion cord of the endoscope may be optimized.

Flexible endoscopes with a bending section may be operated by steering cables. The steering cables comprise steering wires running inside wire pipes. The wire pipes are tubes with limited compressibility. The combination of wire pipes and wires form so-called Bowden cables.

In a first aspect of the disclosure an endoscope is provided. In an embodiment according to the first aspect, the endoscope comprises a positioning interface including a steering controller, a first and a second steering cable, each of the first and the second steering cable comprising a wire pipe and a steering wire translatable within the respective wire pipe, the steering controller being connected to the first and the second steering wires; an insertion cord extending distally from the positioning interface and including a bending section, the insertion cord further comprising a camera and a light emitter positioned at a distal end of the insertion cord, the bending section comprising: segments including a proximal bending segment, a distal bending segment and intermediate bending segments, the segments being interconnected by hinges; the proximal bending segment comprising a first and a second wire pipe abutment; wherein the first and second wire pipe abutments are arranged with a first and second transverse offset from a horizontal midplane of the bending section, the transverse offset constituting 25 to 65 % of a radius of the bending section. The transverse offset is a distance measured perpendicularly from the midplane to the center of the abutment. The transverse offset may be to any side of the midplane, but generally the transverse offset of the two wire pipe abutments is to the same side. Providing wire pipe abutments with a transverse offset as specified from the horizontal midplane of the bending section permits optimal use of the available space in the insertion cord and hereby provision of a compact endoscope with minimum outer diameter of the insertion cord. If the bending section is not circular in cross-section the radius is considered the radius of the smallest circle that will enclose the bending section cross-section.

In a variation of the embodiment of the first aspect, the proximal bending segment comprises a steering wire groove extending from a proximal end at or near the wire pipe abutment to a distal end at or near the horizontal midplane of the bending section, and the steering wire passes through the steering wire groove. By near is meant offset by up to ±10% of the radius from the relevant position. Without a steering wire groove as described the wire pipe has to make sharp bends at the proximal bending segment, which may lead to increased friction between the wire pipe and the steering wire. The wire pipe is a normally a helical winding of wire, so the inner surface of the wire pipe is not smooth but instead fluted. The fluted surface increases friction with the steering wire, and especially when the wire pipe has a sharp bend. The steering wire grooves facilitate transition of the wire pipes from a spaced apart plane to a plane at or near the horizontal midplane of the bending section with reduced friction. Thus, the steering wires may be guided in the steering wire grooves extending from a position with a transverse offset to the horizontal midplane of the bending section to the horizontal midplane in gentle curves. A benefit of the reduced friction is further that any noise generated from friction is reduced which enhances the perceived quality of the endoscope.

In another variation of the present embodiment, the steering wire groove is open. Provision of an open steering wire groove facilitates manufacturing in an injection mold. The open groove may be provided by slides removably insertable in the mold.

In an example of the present embodiment, the axial length of the steering wire groove is 1-3 mm, such as 1.5-2.8 mm, e.g. approximately 2.6 mm. The axial length of the steering wire groove may be longer than this, especially for endoscopes having a relatively large outer diameter of e.g. 6 mm, but it is generally not preferred, which is mainly due to that the part with the steering wire groove is inflexible so steerability of the endoscope may be impaired. A shorter length leads to sharper curves of the steering wire grove, which is generally not preferred due to increased friction.

In an example of the present embodiment, the bending section comprises Polyoxymethylene (POM), such as at least 65% by weight of the bending section is comprised of POM. Other materials are conceivable, such as other polymer materials or metals. For single-use endoscopes it is, however, generally preferred to make the bending section of a polymer material in view of cost and carbon footprint. Alternative materials include for example polypropylene (PP) and methyl methacrylate acrylonitrile butadiene styrene (M-ABS). POM has a low friction coefficient and excellent wear resistance, which is advantageous for the bending section exposed to friction and wear by the steering wire. Low friction of contact between the steering wire and the bending section provides excellent maneuverability of the endoscope and ease of operation of the controller of the positioning interface.

In an example of the present embodiment, the distal and intermediate bending segments, and hinges, are comprised in a one-piece part and the proximal bending segment is a separate part. Providing the proximal bending segment as a separate part may be beneficial in that the proximal bending segment may be made from a different material than the rest of the bending section and hence optimized for the purpose. Further it may facilitate assembly of the endoscope and threading of the steering wire through the bending section.

In an alternative example of the present embodiment, the bending section is a one-piece fused polymer part comprising the proximal bending segment, intermediate bending segment, distal bending segment, and hinges. Making the bending section as an integral construction, e.g. by injection molding, provides for cost efficient manufacturing and assembly. Providing the bending section as a one-piece part reduces the potential risk of mistakes in assembly.

In a variant of the present embodiment, the first wire pipe abutment is arranged at a first transverse offset and the and the second wire pipe abutment is arranged at a second transverse offset from the horizontal midplane of the bending section. The possibility of arranging the wire pipe abutments at different transverse offsets provides freedom to utilize the space in the insertion cord to the maximum, thereby providing a very compact construction with minimum outer diameter of the insertion cord.

In an example of the present embodiment, a wire pipe abutment center of the wire pipe is arranged at an angle from a bending section center with respect to the horizontal midplane, where the angle is in the interval of 10 to 75 degrees, such as 15 to 55 degrees. The wire pipe abutment center may be positioned at any suitable angle from the bending section center, such as a larger angle of up to nearly 90 degrees is possible. A large angle of nearly 90 degrees would provide that the wire pipes are positioned out of the way leaving space for other parts and potentially providing a very compact construction. With the large angle the travel of the steering wire and the bends thereof increase, which could increase friction and is considered to outweight the advantage of having a very compact construction and hence this is considered less preferable.

In an example of the present embodiment, the bending section has a diameter in the interval of 1.8 mm to 6.5 mm, such as in the interval of 2.0 mm to 5 mm, such as 2.5 mm. A bending section with a diameter in these intervals would be suitable for e.g. bronchoscopes or rhinolaryngoscopes. The disclosure is also relevant for endoscopes with a larger bending section diameter, but it is considered most relevant for endoscopes with relatively small diameter, where the available space in the insertion cord is most strained.

In an example of the present embodiment, the proximal bending segment comprises a steering wire groove extending from the first wire pipe abutment to a distal end opening at a distal end of the proximal bending segment, and wherein the distal end opening is traversed by the horizontal midplane or arranged at a transverse offset from the horizontal midplane of less than 25% of the radius of the bending section.

In an example of the present embodiment, the proximal bending segment comprises a first steering wire groove extending from the first wire pipe abutment to a first distal end opening at a distal end of the proximal bending segment, wherein the proximal bending segment comprises a second steering wire groove extending from the second wire pipe abutment to a second distal end opening at the distal end of the proximal bending segment, wherein the first steering wire groove and the second steering wire groove are located on opposite sides of an orthogonal midplane that is orthogonal to the horizontal midplane, wherein the first distal end opening is traversed by the horizontal midplane or arranged at a transverse offset from the horizontal midplane of less than 25% of the radius of the bending section, wherein the second distal end opening is traversed by the horizontal midplane or arranged at a transverse offset from the horizontal midplane of less than 25% of the radius of the bending section, wherein the first wire pipe abutment comprises a recess in a proximal end of the proximal bending segment, and wherein the second wire pipe abutment comprises a recess in the proximal end of the proximal bending segment.

In an example of the present embodiment the transverse offset of the first wire pipe abutment is different from the transverse offset of the second wire pipe abutment.

In an example of the present embodiment, the transverse offset of the first wire pipe abutment is different from the transverse offset of the second wire pipe abutment, wherein the first steering wire groove comprises an open channel extending from the first wire pipe abutment to the first distal end opening, and wherein the second steering wire groove comprises an open channel extending from the second wire pipe abutment to the second distal end opening.

A second aspect of the disclosure relates to a visualization system. In one embodiment, the visualization system comprises: an endoscope according to the first aspect; and a video processing apparatus configured to communicatively connect with the endoscope to receive a video stream therefrom.

One or more objects may be met by aspects of the present invention described in the following embodiments, variations and examples thereof.

A person skilled in the art will appreciate that any one or more of the above embodiments, variations and examples thereof may be combined with any one or more of the other embodiments, variations and examples thereof. For example, the wire pipe abutments can be on a common plane or can have different transverse offsets, and the steering wire grooves can be open or closed, and these features can be arranged in different combinations that are not mutually exclusive.

### BRIEF DESCRIPTION OF THE DRAWINGS

Embodiments of this disclosure will be described in more detail below with reference to the accompanying figures. The figures illustrate embodiments, variations and examples to facilitate the understanding of a person of ordinary skill in the art and are not to be construed as limiting the scope of the invention.
FIG. 1 is an illustration of an endoscope and a visualization system according to the detailed description;
FIG. 2 schematically illustrates an example of a distal tip housing and bending section;
FIG. 3 is a side view of an bending section according to the detailed description;
FIG. 4 is a perspective view of the bending section of FIG. 3;
FIG. 5 is an enlarged detail view of a proximal portion of the bending section of FIG. 4, partly transparent for purpose of illustration;
FIG. 6A illustrates a portion of the bending section of Fig. 4 and position of a cross-sectional view I-I;
FIG. 6B illustrates cross-sectional view I-I of FIG. 6A;
FIG. 7A illustrates a portion of the bending section of FIG. 4 and position of a cross-sectional view II-II;
FIG. 7B illustrates cross-sectional view II-II of FIG. 7A;
FIG. 7C illustrates a cross-sectional view corresponding to FIG. 7B of a variant of the bending section;
FIG. 8A illustrates a portion of the bending section of FIG. 4 and position of a longitudinal view III-III;
FIG. 8B illustrates longitudinal section III-III of FIG. 8A;
FIG. 9A illustrates a portion of the bending section of FIG. 4 and position of a cross-sectional view IV-IV;
FIG. 9B illustrates cross-sectional view IV-IV of FIG. 9A;
FIG. 9C illustrates a cross-sectional view corresponding to FIG. 9B of a variant of the bending section;
FIG. 10 illustrates a proximal bending section part according to another variant;
FIG. 11 illustrates a proximal bending section part according to another variant; and
FIG. 12 illustrates a proximal bending section part according to a further variant.

### DETAILED DESCRIPTION

The term "distal," as used herein, refers to a direction or position that is generally towards a target site, and the term "proximal," as used herein, refers to a direction or position that is generally away from the target site.

Unless otherwise defined, all technical and scientific terms used herein have the same meaning as commonly understood by one of ordinary skill in the art. In case of conflict, the present document, including definitions, will control. Preferred methods and materials are illustrated below, although apparatuses, methods, and materials similar or equivalent to those illustrated herein may be used in practice or testing. The materials, methods, and examples disclosed herein are illustrative only and not intended to be limiting.

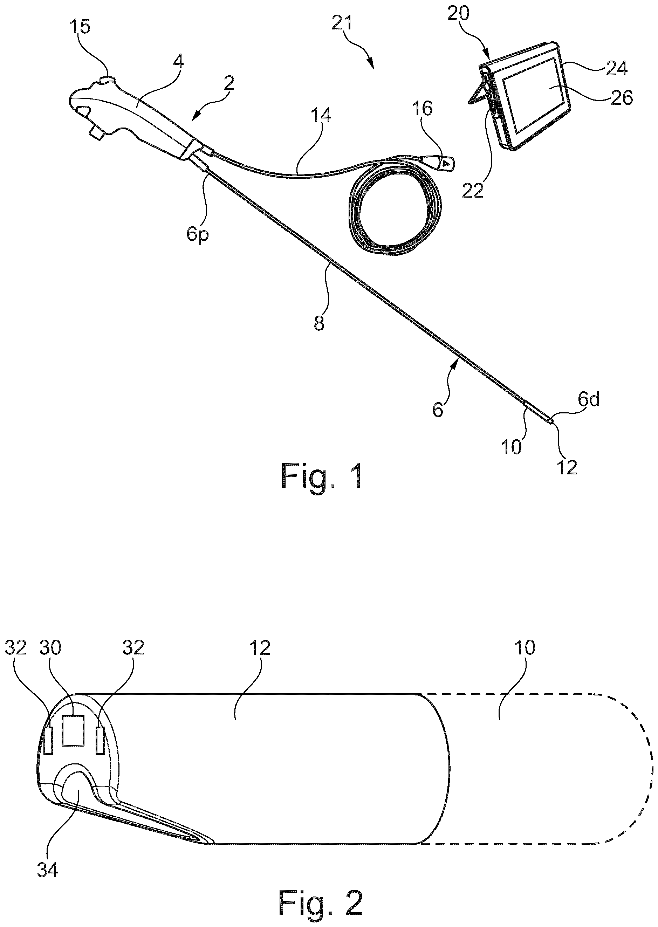

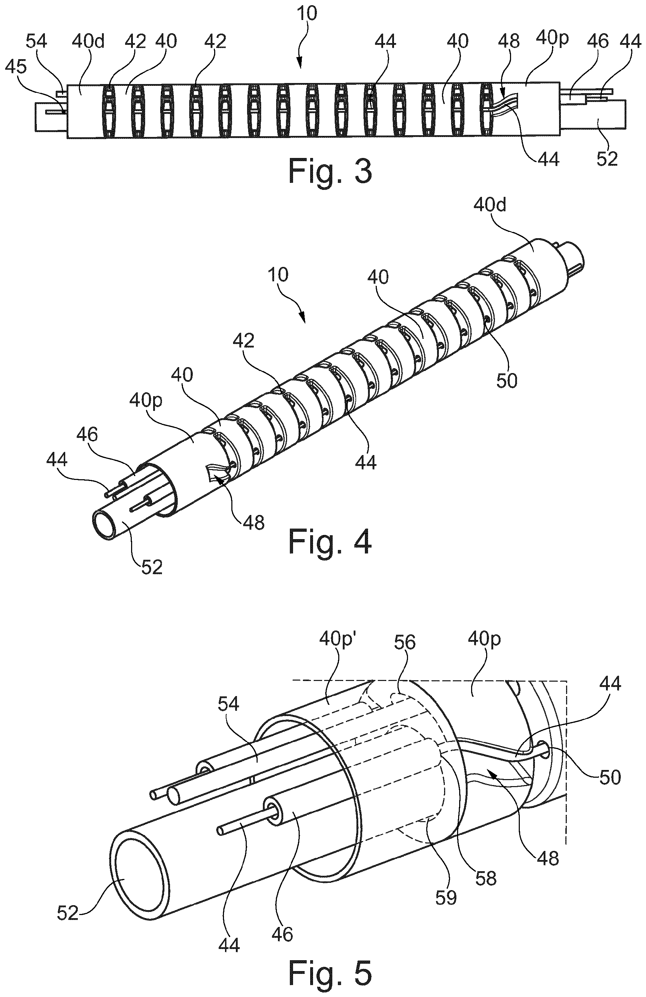

FIG. 1 is an illustration of an endoscope 2 comprising a positional interface exemplified by a handle 4. The endoscope 2 comprises an insertion cord 6 having a proximal end 6p and a distal end 6d, the insertion cord 6 including an insertion tube 8 and a bending section 10. A distal tip comprising a tip housing 12 extends from the bending section 10 as will be discussed in more detail with reference to FIG. 2. The bending section 10 may comprise a single-piece polymeric structure as will be discussed in further detail with reference to FIG. 3. The bending section 10 may be controlled to bend in two directions by operating a controller, such as a lever 15 at the handle 4. The lever 15 may be connected to the bending section 10 by steering wires as will be described in more detail below. The endoscope 2 is connectable to a video processesing apparatus 20 thereby forming a visualization system 21. Connnection of the endoscope 2 to the video processesing apparatus 20 may as illustrated be via an umbilical cord 14 comprising a cable connector 16 insertable in a cable connector receptor 22. The video processing apparatus 20 comprises a processor 24. The video processesing apparatus 20 may as shown here include a housing supporting a display screen 26 connected to the processor 24 and operable to present images provided by the processor 24. Alternatively or supplementary the video processing apparatus 20 may be connectable to a separate display screen (not shown).

An example of the distal tip housing 12 is illustrated in more detail in FIG. 2. The illustrated distal tip housing 12 comprises a camera 30 and light emitters 32 as well as an working channel opening 34. Example light emitters 32 include light emitting diodes (LEDs), organic LEDs (OLEDs), laser diodes, etc., which may be arranged in the distal tip housing. Alternatively, the light emitters 32 may be arranged in the handle or in a separate box and connected to the distal tip housing by optical fibers. The distal tip housing 12 may be a 2K construction made of fused polymer components. The proximal end of the distal tip housing 12 is connected to the bending section 10 as schematically illustrated.

FIGS. 3 and 4 illustrate an embodiment of the bending section 10 comprising a plurality of segments 40 intermediate a proximal segment 40p and a distal segment 40d which in turn is connected to the distal tip housing 12. The segments are interconnected by polymeric strips 42, or hinges, which form part of a one-piece structure and bend upon tensioning of one or the other of the steering wires 44, which run through the bending section from the proximal segment 40p to the distal segment 40d, where the steering wires are attached or fixed, e.g. by a drop of glue 45 as schematically illustrated. The steering wires 44 each run through a respective wire pipe 46 extending from the handle to the proximal segment 40p. The wire pipes 46 abut the proximal segment 40p and the naked steering wires run through a steering wire groove 48 arranged in the proximal segment 40p and through wire openings 50 in the segments 40. By "naked steering wire" it is meant a steering wire portion that is not surrounded by the wire pipe. A working channel tube 52 is arranged in the insertion cord 6 to extend from the handle to the distal tip housing 12. The working channel tube 52 can, for example, be used for advancing tools through the endoscope or to provide suction at a site in the body, e.g. to remove blood or secretion. Cables 54 for the camera and light emitters run through the insertion cord 6 from the handle to the distal tip housing 12. The wire pipes 46 may abut a most proximal surface of the proximal end segment or extend into a recess in the proximal end segment, but the wire pipes 46 should not extend into the steering wire grooves.

The steering wires may be two individual steering wires or the steering wires may be made up by one single wire running from the controller to the distal end of the bending section at one side thereof and back to the controller at the other side of the bending section. In the latter case the steering wires are in this disclosure considered individual wires even though they are two stretches of the same wire.

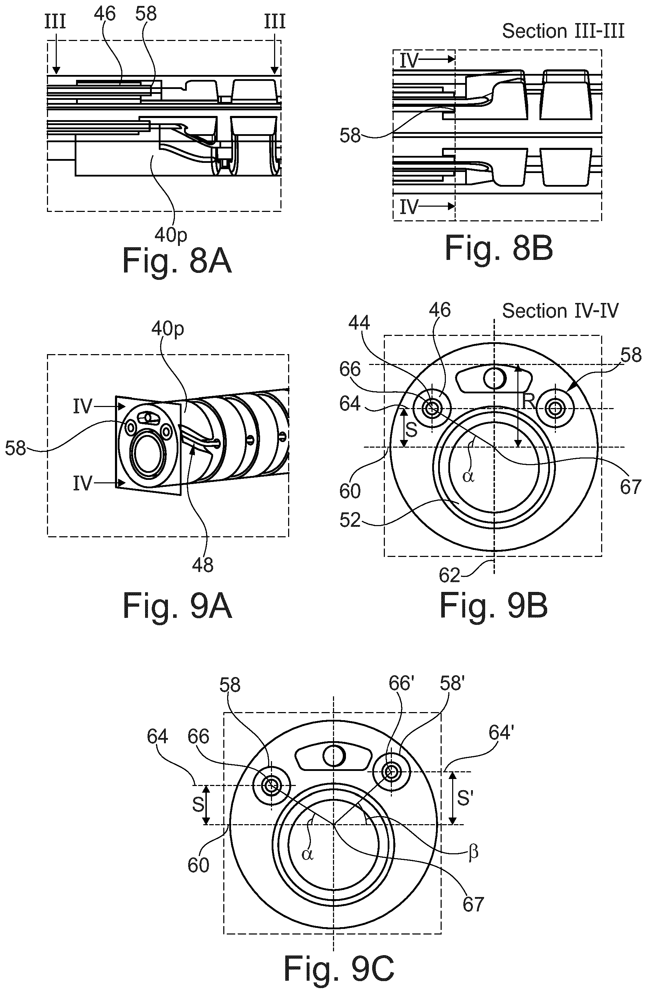

More details can be seen in FIG. 5, which is an enlarged view of the proximal part of the bending section. For purpose of illustration a collar portion 40p' of the proximal segment 40p is transparent to show details otherwise hidden. The collar portion 40p' may be used for attachment of an insertion tube (not shown), e.g. by fitting the insertion tube inside the collar portion 40p' and potentially fix the insertion tube to the collar portion 40p', e.g. by adhesive. In this example, the wire pipe 46 terminates where it abuts the proximal segment 40p at a wire pipe abutment 58 (see also FIGS. 8A and 8B) at the proximal segment 40p, whereas the steering wire 44 continues into the steering wire groove 48 and via wire openings 50 through the bending section to the distal end thereof. The cables 54 run through a cable opening 56 in the proximal segment 40p and through corresponding cable openings in the other segments. The working channel tube 52 runs through a tube opening 59 in the proxial segment 40p and through corresponding tube openings in the other segments. Cable openings 56 and tube openings 59 may be connected to form one single lumen (not shown).

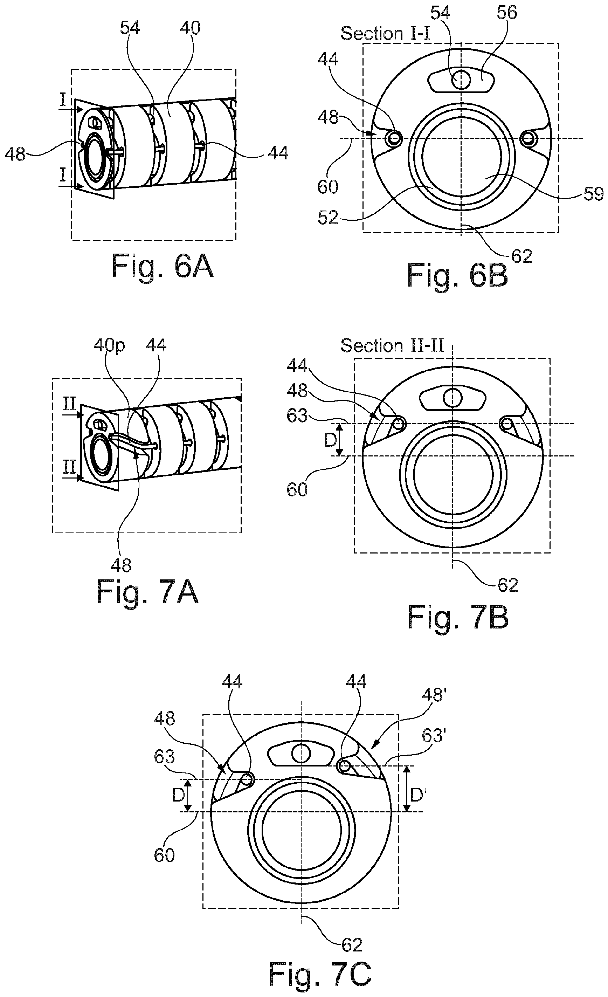

A portion of the bending section is illustrated in FIG. 6A showing the position of a cross-section I-I, which is orthogonal to a longitudinal axis of the bending section and is shown in FIG. 6B. The cross-section is taken at the distal end of the groove 48 in the proximal segment 40p. The bending section 10 has a horizontal midplane 60, and at the distal end of the proximal segment 40p the steering wire groove 48 is arranged at or near the horizontal midplane 60 of the bending section, so the steering wire 44 arranged in the steering wire groove here is at or near the horizontal midplane 60. When one or the other steering wire 44 is pulled the bending segments will bend to a respective side of the vertical midplane 62 of the bending section. The cables 54 are arranged in the cable opening 56, and the working channel tube 52 is arranged in the tube opening 59. The midplane traverses the longitudinal axis, which in this example is at the center of the circular cross-section.

Similarly a portion of the bending section is illustrated in FIG. 7A showing position of a cross-section II-II, which is shown in FIG. 7B. Cross-section II-II is taken at the proximal end of the steering wire groove 48 in the proximal segment 40p. The steering wire 44 is arranged in the steering wire groove 48 and is here at an elevated steering wire level 63 at a distance D from the horizontal midplane 60. The steering wire groove 48 in the embodiment shown is open to the side and provided with suitable roundings and slip angles to facilitate molding, e.g. by injection molding in a polymer material, such as POM. In an injection molding process the grooves 48 may be formed by slides (not shown).

The bending section cross section may be non-symmetrical as illustrated in FIG. 7C, which illustrates a cross-sectional view corresponding to FIG. 7B of a variant of the bending section. Here the steering wire grooves 48 are not identical. One steering wire groove 48 is arranged at the elevated steering wire level 63 at the distance D from the horizontal midplane 60, whereas the other steering wire groove 48' is arranged at an elevated steering wire level 63' at the distance D' from the horizontal midplane 60. The term "elevated" is used with reference to the figure, where the cable opening is above the midplane, thus the steering wire level is on the side of the cross-section where the cable opening is located. With the non-symmetrical arrangement facilitates arrangement of extra equipment, such as an extra tube for fluid supply or suction, in the insertion cord and increases flexibility in design of the endoscope. Such an extra tube is for example used in in ureteroscope with active flushing.

A portion of the bending section is illustrated in FIG. 8A showing position of a longitudinal section III-III, which is shown in FIG. 8B. The longitudinal section is taken at the distal end of the wire pipe 46 showing the wire pipe abutment 58 at the proximal segment 40p. The illustrated abutment 58 is a recess into which the wire pipe 46 may be inserted, and a drop of glue may be added at the recess to fix the wire pipe 46.

FIGS. 8B and 9A further illustrate a position of a cross-section IV-IV, which is shown in FIG. 9B. The cross-section IV-IV is taken at the wire pipe abutment 58 in the proximal segment 40p. The two abutments 58 are positioned at a wire pipe level 64, where the wire pipe abutment center 66 at the abutment 58 is arranged at a transverse offset S from the horizontal midplane 60. The wire pipe abutment center 66 is arranged at an angle α from the bending section center 67 with respect to the horizontal midplane 60. Having the wire pipes 46 arranged at the transverse offset S from the horizontal midplane 60 leaves more space for the insertion tube 52.

The bending section cross section may be non-symmetrical as illustrated in FIG. 9C, which illustrates a cross-sectional view corresponding to FIG. 9B of a variant of the bending section. In this variant one abutment 58 is positioned at the wire pipe level 64, whereas the other abutment 58' is positioned at a wire pipe level 64' with wire pipe abutment center 66' at a transverse offset S' form the horizontal midplane 60. The wire pipe abutment center 66' is arranged at an angle β from the bending segment center 67 with respect to the horizontal midplane 60. A non-symmetrical layout may be advantageous in some cases and thereby providing more variability in the construction and exploit of the available space of the cross-section to fit wires, cables etc.

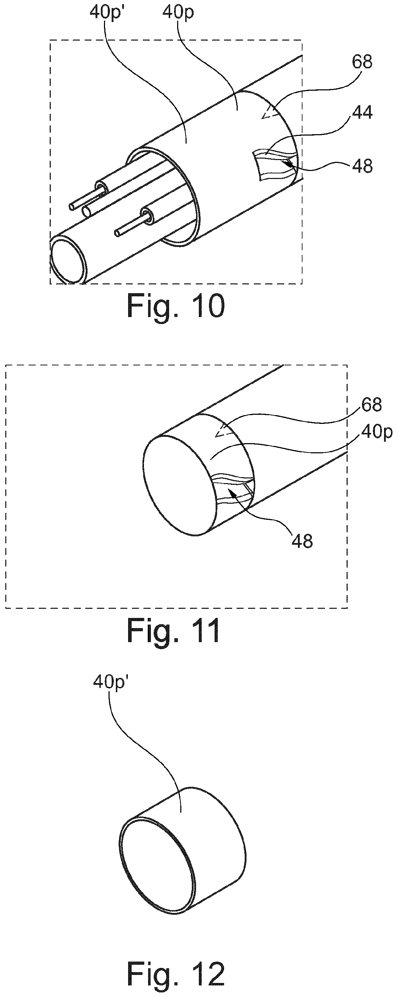

The bending section could be molded in one piece as depicted, for example, in FIG. 3 and 4. As an alternative, the bending section can be made up of individual parts. As an example, the proximal segment 40p with the collar portion 40p' could be one part as illustrated in FIG. 10. The proximal segment 40p may connect to or register with the remaining bending section, for example by provision of a cut-out 68 to register with a protrusion (not shown) of the bending section for alignment of the parts. This may provide more freedom in designing and producing the part with the steering wire groove 48 for the steering wire 44 than if the part was integrally formed in one-piece with the other segments of the bending section. FIG. 11 illustrates the proximal segment 40p as a single part, i.e. without the collar portion 40p'. The collar portion 40p' may in some cases be dispensed with e.g. if the collar portion is not relevant in a particular construction. Again, the proximal segment 40p may connect to or register with the remaining bending section, for example by provision of a cut-out 68 to register with a protrusion (not shown) of the bending section for alignment of the parts. The collar portion 40p' may also be a separate part as illustrated in FIG. 12.

A positioning interface functions to control the position of the insertion cord. A handle is an example of a positioning interface and, unless stated otherwise, the terms are used interchangeably. The handle also functions to provide the steering control, e.g. knobs, levers, buttons, and the like, to steer the field of view of the camera. Alternatively, a different positioning interface can be provided that is connected to the insertion cord and is detachably connected to a robotic arm. The insertion cord thus extends from the robotic arm, and the intrusive medical device is detachable from the robotic arm. The robotic arm responds to signals, including voice commands from an operator, to rotate, translate, and otherwise position the proximal end of the insertion cord, as an operator would do manually. The positioning interface can include control actuators, including manual control actuators. Alternatively or additionally, control actuators can be provided in or on the robotic arm or by the robotic system including the robotic arm, thereby potentially reducing the cost of the intrusive medical device. Example control actuators include single axis actuators, including linear motion actuators. A linear motion actuator may comprise a threaded rod coupled to a threaded nut portion, in which a motor rotates the rod to translate the nut portion.

A sleeve or bending cover (not shown) may be provided over the bending section 10 to fluidly seal the spaces between adjacent segments 40.

Variations of the present embodiment may be combined to form additional variations of the embodiment.

In device claims enumerating several means, several of these means can be embodied by one and the same hardware components. The mere fact that certain measures are recited in mutually different dependent items or described in different embodiments does not indicate that a combination of these measures cannot be used to advantage.

The terms "first," "second," and the like in the description and in the claims, if any, are used for distinguishing between similar elements and not necessarily for describing a particular sequential or chronological order. It is to be understood that any terms so used are interchangeable under appropriate circumstances such that the embodiments described herein are, for example, capable of operation in sequences other than those illustrated or otherwise described herein.

The term "comprises/comprising" are generally interpreted to be open transition terms which specify the presence of stated features, integers, steps or components but do not preclude the presence or addition of one or more other features, integers, steps, components or groups thereof. The terms "consisting of" or "consists of" are closed transition terms, and include only the components, structures, steps, or the like specifically listed in conjunction with such terms, as well as that which is in accordance with patent law.

### Reference numerals:

| REFERENCE NUMERALS: | |
|---|---|
| 2 | Endoscope |
| 4 | Handle |
| 6 | Insertion cord |
| 6p | Proximal end |
| 6d | Distal end |
| 8 | Insertion tube |
| 10 | Bending section |
| 12 | Tip housing |
| 14 | Umbilical cord |
| 15 | Lever |
| 16 | Cable connector |
| 20 | Video processing apparatus |
| 21 | Visualization system |
| 22 | Cable connector receptor |
| 24 | Processor |
| 26 | Display screen |
| 30 | Camera |
| 32 | Light emitters |
| 34 | Working channel opening |
| 40 | Segment |
| 40p | Proximal segment |
| 40p' | Collar portion |
| 40d | Distal segment |
| 42 | Strip (hinge) |
| 44 | Steering wire |
| 45 | Glue |
| 46 | Wire pipe |
| 48 | Steering wire groove |
| 50 | Wire opening |
| 52 | Working channel tube |
| 54 | Cables |
| 56 | Cable opening |
| 58 | Wire pipe abutment |
| 59 | Tube opening |
| 60 | Horizontal midplane |
| 62 | Vertical midplane |
| 63 | Elevated steering wire level |
| 63' | Elevated steering wire level |
| 64 | Wire pipe level |
| 64' | Wire pipe level |
| 66 | Wire pipe abutment center |
| 66' | Wire pipe abutment center |
| 67 | Bending section center |
| 68 | Cut-out |
| α | Angle |
| β | Angle |
| D | Distance |
| D' | Distance |
| R | Radius |
| S | Transverse offset |
| S' | Transverse offset |

## Claims

1. An endoscope comprising:
a positioning interface including a steering controller, a first and a second steering cable, each of the first and the second steering cable comprising a wire pipe and a steering wire translatable within the respective wire pipe, the steering controller being connected to the first and the second steering wires;
an insertion cord extending distally from the positioning interface and including a bending section, the insertion cord further comprising a camera and a light emitter positioned at a distal end of the insertion cord, the bending section comprising:
segments including a proximal bending segment, a distal bending segment and intermediate bending segments, the segments being interconnected by hinges;
the proximal bending segment comprising a first and a second wire pipe abutment;
wherein the first and second wire pipe abutments are arranged with a first and second transverse offset from a horizontal midplane of the bending section, the transverse offset constituting 25 to 65 % of a radius of the bending section.

2. The endoscope of claim 1, wherein the proximal bending segment comprises a steering wire groove extending from a proximal end at or near the wire pipe abutment to a distal end at or near the horizontal midplane of the bending section, and the steering wire passes through the steering wire groove.

3. The endoscope of claim 2, wherein the steering wire groove is open.

4. The endoscope of claim 2 or 3, wherein an axial length of the steering wire groove is 1-3 mm, such as 1.5-2.8 mm, e.g. approximately 2.6 mm.

5. The endoscope of one of the preceding claims, wherein the bending section comprises Polyoxymethylene (POM), such as at least 65% by weight of the bending section is comprised of POM.

6. The endoscope of one of the preceding claims, wherein the distal and intermediate bending segments, and hinges, are comprised in a one-piece part and the proximal bending segment is a separate part.

7. The endoscope of one of claims 1-5, wherein the bending section is a one-piece fused polymer part comprising the proximal bending segment, intermediate bending segment, distal bending segment, and hinges.

8. The endoscope of one of the preceding claims, wherein the first wire pipe abutment is arranged at a first transverse offset and the and the second wire pipe abutment is arranged at a second transverse offset from the horizontal midplane of the bending section.

9. The endoscope of any one of the preceding claims, wherein a wire pipe abutment center of the wire pipe is arranged at an angle from a bending section center with respect to the horizontal midplane, where the angle is in the interval of 10 to 75 degrees, such as 15 to 55 degrees.

10. The endoscope of one of the preceding claims, wherein the bending section has a diameter in the interval of 1.8 mm to 6.0 mm, such as in the interval of 2.0 mm to 3 mm, such as 2.5 mm.

11. The endoscope of claim 1, wherein the proximal bending segment comprises a steering wire groove extending from the first wire pipe abutment to a distal end opening at a distal end of the proximal bending segment, and wherein the distal end opening is traversed by the horizontal midplane or arranged at a transverse offset from the horizontal midplane of less than 25% of the radius of the bending section.

12. The endoscope of claim 1, wherein the proximal bending segment comprises a first steering wire groove extending from the first wire pipe abutment to a first distal end opening at a distal end of the proximal bending segment, wherein the proximal bending segment comprises a second steering wire groove extending from the second wire pipe abutment to a second distal end opening at the distal end of the proximal bending segment, wherein the first steering wire groove and the second steering wire groove are located on opposite sides of an orthogonal midplane that is orthogonal to the horizontal midplane, wherein the first distal end opening is traversed by the horizontal midplane or arranged at a transverse offset from the horizontal midplane of less than 25% of the radius of the bending section, wherein the second distal end opening is traversed by the horizontal midplane or arranged at a transverse offset from the horizontal midplane of less than 25% of the radius of the bending section, wherein the first wire pipe abutment comprises a recess in a proximal end of the proximal bending segment, and wherein the second wire pipe abutment comprises a recess in the proximal end of the proximal bending segment.

13. The endoscope of claim 12, wherein the transverse offset of the first wire pipe abutment is different from the transverse offset of the second wire pipe abutment.

14. The endoscope of claim 12, wherein the transverse offset of the first wire pipe abutment is different from the transverse offset of the second wire pipe abutment, wherein the first steering wire groove comprises an open channel extending from the first wire pipe abutment to the first distal end opening, and wherein the second steering wire groove comprises an open channel extending from the second wire pipe abutment to the second distal end opening.

15. A visualization system comprising:
the endoscope of any one of the preceding claims; and
a video processing apparatus configured to communicatively connect with the endoscope to receive a video stream therefrom.
